# EUROPEAN PATENT SPECIFICATION

(11) **EP 1 513 442 B1**
(45) Date of publication and mention of the grant of the patent: **16.01.2008**
(21) Application number: 03738144.9
(22) Date of filing: 17.06.2003
(51) Int. Cl.: A61B 3/16

(54) **AN APPARATUS FOR MEASURING INTRAOCULAR PRESSURE**
GERÄT ZUR MESSUNG DES AUGENINNENDRUCKS
DISPOSITIF SERVANT A MESURER LA PRESSION INTRA-OCULAIRE

(30) Priority: 17.06.2002 FI 20021172
(43) Date of publication of application: 16.03.2005
(73) Proprietor: Tiolat Oy, 00660 Helsinki (FI)
(72) Inventor: KONTIOLA, Antti, FIN-00660 Helsinki (FI)
(74) Representative: Söderman, Päivi Karin Lisbeth
(86) International application number: PCT/FI2003/000489
(87) International publication number: WO 2003/105680

(56) References cited:
- EP-A2- 1 121 895
- WO-A1-96/06560
- US-A- 5 176 139
- US-A- 5 865 742
- US-A- 6 093 147
- US-B1- 6 394 954

## Description

The present invention relates to an apparatus for measuring intraocular pressure. The apparatus is based on an understanding of the laws governing the impact of an object with the eye.

Intraocular pressure is generally measured using a tonometer, which is placed on the surface of the cornea and which measures its elasticity using various methods (Goldmann's tonometer, Schiötz's tonometer, etc.). Two of the most commonly used principles are the measurement of the force required to applanate a certain area of the surface of the eye, or the measurement of the diameter of the area that is applanated by a known force. These methods require the patient's co-operation and cannot be applied without general anaesthesia to small children, persons suffering from dementia, or animals.

Methods, such as US patent publications, 5148807, 5279300, and 5299573, in which the surface of the cornea is not touched, the intraocular pressure being measured instead with the aid of a water or air jet, or various kinds of waves, have also been developed. These methods are technically complex and thus expensive. Meters operating on the air-jet principle are widely used by opticians, but their cost has prevented them from being more extensively used by general practitioners.

US patent publication 5176139 also discloses a method, in which a freely-falling ball is dropped onto the eyelid and the height of the ball's rebound is measured. It is also known from Finnish patent application 973094 an apparatus which is based on the fact that a probe is forwarded with a certain speed to contact the surface of the eye, wherefrom it will rebounded. The movements of the probe can be the basis for calculation of the intraocular pressure.

Document US-A-6 093 147 discloses an apparatus for measuring intraocular pressure according to the preamble of claim 1.

The present invention is intended to achieve certain improvements specifically in the latter quite good apparatus. The intention is also, however, to retain the apparatus's simple, economical, and precisely measuring basic construction, by means of which intraocular pressure can also be measured in patients incapable of co-operating, who can be restrained only momentarily. In addition, the meter is also suitable for extensive screening campaigns, as measurement is rapid and requires neither a local anaesthetic nor specially trained operators. It is also intended to permit home monitoring for patients with intraocular pressure complaints.

The above and other benefits and advantages of the present invention are achieved by using an apparatus according to the invention which is defined in the accompanying Claims.

In the following, the invention is described with reference to the accompanying drawings, in which certain well regarded properties of the invention are presented.

Thus:
Figure 1 shows one version of a practical apparatus, in order to provide a general view of it;
Figure 2 shows a vertical cross section of the above, rotated to an angle of 90 degrees to the above;
Figure 3 shows a more detailed picture of the essential component of the invention, by means of which the probe is launched towards the eye.

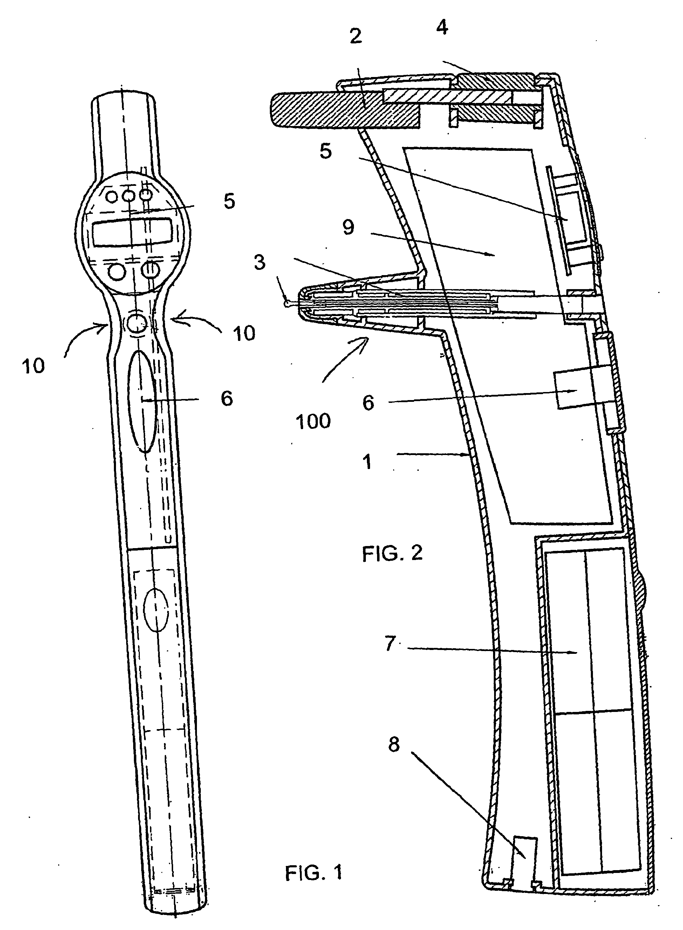

Thus, Figure 1 shows, as stated above, one practical embodiment of an apparatus applying the principle according to the invention, while Figure 2 shows a cross section of it. These two figures can be used together to illustrate the general principle and construction.

The apparatus is formed of a case component 1 made of a suitable material, inside of which all the components that are essential for measurement are fitted.

The case or body component 1 is essentially elongated and includes at its upper end a forehead support 2, which is intended to adjust the distance from which the probe 3 impacts the eye being measured. The forehead support 2 is specifically adjustable, one comfortable way of arranging adjustability being to use an adjustment wheel 4, which can be easily rotated with a finger.

The apparatus includes a display and control component 5, which is particularly a liquid-crystal panel, in which the measurement result is displayed, and the related control buttons, etc, which are required at various times. Reference number 6 marks the operating switch, which, when pressed, releases the probe 3 towards the eye.

Operating power for the apparatus comes from dry cells or batteries 7, while the apparatus can additionally have a socket 8, to which an external recharging device or power supply can be connected.

The electronics of the apparatus according to the invention are assembled on a circuit board 9, which is shown schematically.

Figure 1 clearly shows one property of the apparatus according to the invention that makes it more comfortable and accurate to use. This is the narrowed part, incorporated in the apparatus next to the probe 3. These narrowings are marked with the reference number 10. They are intended to allow patients performing self measurement to be able to see the coloured part of the eye through a mirror, on either side of the narrowing, so that it is easy for them to use this to align the measuring apparatus accurately, thus ensuring that the probe will strike the desired point on the surface of the eyeball. As can be seen, the narrowings 10 are located next to the probe 3.

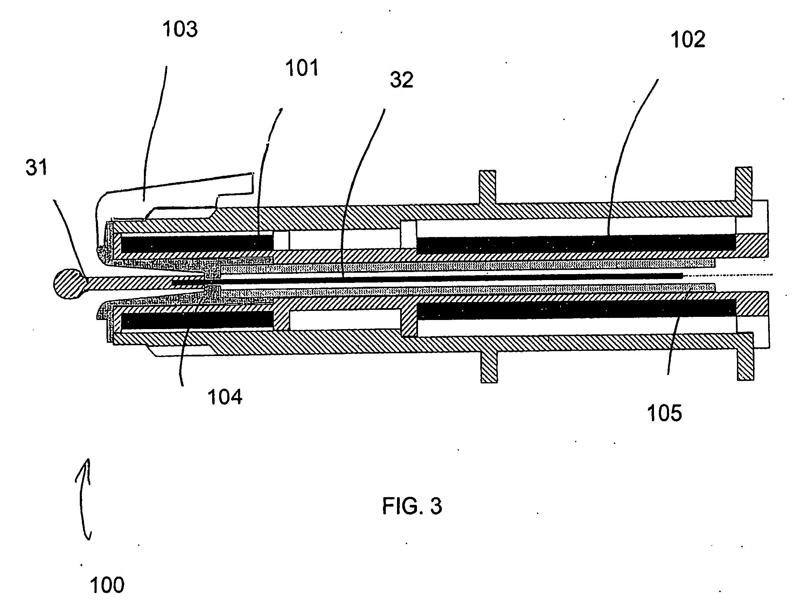

Figure 3 shows the totality, marked with the reference number 100, relating to the launch of the probe of the apparatus according to the invention, the recording of the measurement, and other similar operations. Thus, according to one well regarded embodiment, the totality 100 includes two coils 101 and 102, the frontmost of which is intended to provide the probe 3 with the necessary velocity. The rearmost coil 102, on the other hand, is related to various measurement and settings functions.

An inner tube 105 is held in place by an inner sleeve 104, which lies around the probe and is secured by means of a separate plug 103 particularly equipped with a screw attachment. The inner sleeve and the inner tube can be changed. Naturally, the probe 3 can be changed while over time the inner sleeve and inner tube may also collect enough extraneous material for this to interfere with measurement in an apparatus with such small tolerances. As cleaning the sleeve and tube is difficult, it will then be easier to change them. If desired, the inner sleeve and the inner tube can be manufactured as a single integrated component.

According to one well regarded embodiment, the probe can be formed of a non-magnetic point part 31 and a shaft part 32 of steel or other similar material.

The point part 31 is preferably manufactured from a plastic material, for reasons of both manufacturing technique and manufacturing cost. Another characteristic of the probe is that a shoulder is formed where the point joins the shaft and is made to rest on the edges of the opening of the inner sleeve 104, through which the shaft part 32 runs. This positions the probe very precisely for the start of the measurement event. For reasons of hygiene, the point part of the probe is generally clearly outside the apparatus in the starting position.

The non-magnetic point part of the probe is in such a ratio to the magnetic shaft part that the shaft extends for a certain distance inside the coil 101, preferably, for example, almost halfway into the coil, counting from its front, as shown in Figure 3. The voltage fed to the coil induces a pushing force in the probe, causing it to move towards the eye.

Earlier, the probe had the problem that, under certain conditions, the eyelashes tended to catch on the front of the probe. The point part of the probe has now been given a very round shape, thus preventing catching.

The simplified operation of the apparatus is as follows. Power is supplied to the front coil 101, causing the probe to begin moving and to impact the eye. The probe 3 rebounds and the movements, which take place in a manner depending on the intraocular pressure, are recorded with the aid of the rear coil 102, taken to a suitable data-processing unit to be processed particularly by a microprocessor, and the result is displayed by the display device 5. The coil 101 can operate in pulses, or power can be connected to the coil for the entire duration of the measurement, so that the power is cut off only when it is intended to pull the probe back inside.

The stability of the measurement is adjusted in many ways, for example, by triggering the movement of the probe always using a constant voltage, which does not alter when the voltage of the dry cells drops. The constant voltage can be adjustable, making it possible to adjust the launch speed of the probe. The starting position of the probe 3 is also made constant in a suitable manner, for example, by having the shaft of the probe rest on its starting position in the inner sleeve 104. One way of ensuring the starting position is described above. Other ways can also be used.

Many different ways can be used to ensure that the probe remains stationary. One example is that when the power to the apparatus is switched on, the rear, measuring coil 102 holds the probe stationary by magnetic force. A function that throws the probe out after measurement, can also be added to the apparatus. This can particularly be performed manually, by pressing a button. The retaining forces need not be continuous, instead, the apparatus can include a detection function, which, for instance, which monitors the probe's position and pulls a probe, which is trying to detach itself, back into place only when necessary.

As described above, the voltage fed to the coil 101 to perform the actual measurement causes the probe to start moving. The rear coil 102 detects the speed of the probe continuously, the general principle being that the probe accelerates rapidly at the start, then, as the journey continues, a change in the voltage/current supplied to the coil reduces the acceleration to a constant, relatively low level, intended to maintain even motion. The input voltage/current of the coil 101 is preferably on for essentially the whole time that the probe proceeds towards the eye and impacts it, but can also be cut off at, or near to, the moment of impact.

According to yet another preferable embodiment, immediately after the impact has occurred and the measurement result has been obtained, the rear coil 102 is activated and pulls the probe back to the starting position. This ensures that the probe does not detach due to the movements of the meter or for other reasons, but can be cleanly removed from its place and thrown into the garbage.

The retraction of the probe can also be activated on the basis of time. This is because, if the probe has not struck the measurement object within a predetermined time, it can be assumed that the measurement has failed, in which case the retraction is activated.

As stated, the core of the apparatus according to the invention is a microprocessor. It is easy to use this to also perform other control operations than those referred to above. In a known manner, the microprocessor can have the task of monitoring the measurement results and making error messages according to how a measurement result differs from the values to which the system should tend, according to data recorded for the use of the microprocessor. Monitoring and calculation functions of this kind are usual in microprocessor-based devices.

As the probe is light and the velocities used are low, there is no danger of damage to the eye. The method can also be applied when the eyelid is closed. The low velocity and small mass of the probe eliminate the need to anaesthetize the eye under any measurement conditions. If necessary, the meter can be calibrated with different standards, by comparing the results with those obtained by other methods, or by using experimental methods.

As has clearly been demonstrated by the above, the apparatus according to the invention has several properties, by means of which ease of use, accuracy, and reliability can be improved and maintained. Many variations are possible while remaining within the scope of the inventive idea and the accompanying Claims. For example, instead of the front coil 101 giving the probe 3 launching power, it is also possible alternatively to use the rear coil 102, without this altering the characteristics of the apparatus. The physical properties of the coils are always selected as required.

An alternative variation that can also be presented is one, in which the magnetic-material part of the probe, marked with the reference number 32, remains permanently inside the apparatus and is prevented from coming out, by making its rear end wider, thus preventing it from coming out, or in some other suitable manner. The front part, which attaches to the rear part 32, would then be replaceable, due to the demands of hygiene. If desired, the positioning of the components could be set up to take place from the other side of the apparatus to the probe side. In other ways the construction would correspond to that described above.

Particularly a microprocessor-based apparatus can perform many functions, which have previously been impossible. Thus it is possible to perform corrections to the measurement results obtained, in which different variables are measured and observed. Thus, the measured variables could be the impact time of the probe, the time the probe is stationary, and the rebound time, while it is also possible to observe velocities and decelerations, and make various averages and ratios, etc. of them. One correction method is such in which the intraocular pressure is corrected upwards, in proportion to the inelasticity of the impact.

## Claims

1. An apparatus for measuring intraocular pressure, which includes a probe (3) supported and located in a case (1), a front coil (101) and a rear coil (102), wherein either the front coil (101) or the rear coil (102) is capable of giving the probe a specific velocity, means for processing and displaying measurement data and controlling operations, and an inner sleeve (104) around the probe (3), the probe (3) being formed of a rear part (32) made of a magnetic material and a front part (31), attached to the rear part, made of non-magnetic material and with a round-shaped point, so that the joint between said front part (31) and said rear part (32) is located, at the start of a measurement, inside the front coil (101) and the point of said front part (31) being located outside the front coil (101), **characterized in that** the inner sleeve (104) has edges on an opening through which the rear part runs (32), and **in that** the joint between said front part (31) and said rear part (32) forms a shoulder, which rests on the edges of the inner sleeve (104).

2. An apparatus according to claim 1, **characterized in that** one of said coils (101, 102) for giving said probe (3) a specific velocity operates, when power is switched on in said apparatus, as a retainer for holding said probe in place.

3. An apparatus according to claim 1, **characterized in that** in said case (1) of said apparatus, there is, essentially next to said probe (3), a narrowing (10), which allows that parts of an eye being measured can be seen, in order to ensure accuracy.

4. An apparatus according to any of claims 1 - 3, further **characterized by** an adjustable (4) forehead support (2).

5. An apparatus according to any of claims 1 - 4, further **characterized by** a replaceable inner tube (105), said rear part (32) of said probe (3) being placed in the replaceable inner tube (105), the inner sleeve (104) being replaceable and holding said inner tube (105) in place.

6. An apparatus according to any of claims 1 - 5, further **characterized in that** said shoulder between said front part (31) and said rear part (32) of said probe (3) is located, at the start of a measurement, against an opening in said inner sleeve (104), which receives an edge of said rear part (32) of said probe (3).

7. An apparatus according to any of claims 1 - 6, further **characterized by** means for correcting measurement results, the correction being an algorithm,

8. An apparatus according to any of claims 1 - 7, further **characterized by** means for correcting measurement results in proportion to how much kinetic energy is lost in the impact and rebound of said probe.

## Patentansprüche

1. Vorrichtung zur Messung von Augeninnendruck, die eine Sonde (3) umfasst, die in einem Gehäuse (1) abgestützt und angeordnet ist, eine vordere Spule (101) und eine hintere Spule (102), wobei entweder die vordere Spule (101) oder die hintere Spule (102) die Fähigkeit hat der Sonde eine bestimmte Geschwindigkeit zu verleihen, Mittel fürs Verarbeiten und Anzeigen von Messdaten und Kontrollieren von Funktionen, und eine innere Hülse (104) um die Sonde (3) herum, welche Sonde (3) aus einem hinter Teil (32) aus einem magnetischen Material und einem, am hinteren Teil befestigten vorderen Teil (31) gebildet wird, der aus einem nicht-magnetischem Material besteht und eine rundgeformte Spitze aufweist, so dass der Übergang zwischen dem vorderen Teil (31) und dem hinteren Teil (32) zu Beginn der Messung innerhalb der vorderen Spule (101) und die Spitze des vorderen Teils (31) außerhalb der vorderen Spule (101) positioniert ist, **dadurch gekennzeichnet, dass** die innere Hülse (104) Anschläge an einer Öffnung hat, durch die der hintere Teil (32) verläuft, und dass der Übergang zwischen dem vorderen Teil (31) und dem hinteren Teil (32) eine Schulter bildet, die auf den Anschlägen der inneren Hülse (104) ruht.

2. Vorrichtung nach Patentanspruch 1, **dadurch gekennzeichnet, dass** eine der Spulen (101, 102) zur Verleihung einer bestimmten Geschwindigkeit für die Sonde (3) beim Einschalten des Stroms für die Vorrichtung als Haltevorrichtung dient, um die Sonde auf Position zu halten.

3. Vorrichtung nach Patentanspruch 1, **dadurch gekennzeichnet, dass** das Gehäuse (1) der Vorrichtung im Wesentlichen neben der Sonde (3) eine Einengung (10) aufweist, die es ermöglicht, dass zur Gewährleistung der Genauigkeit Teile eines zu vermessenden Auges gesehen werden können.

4. Vorrichtung nach einem der Patentansprüche 1-3, **gekennzeichnet** des Weiteren durch eine verstellbare (4) Stirnstütze (2).

5. Vorrichtung nach einem der Patentansprüche 1-4, **gekennzeichnet durch** ein austauschbares Innenrohr (105), wobei der hintere Teil (32) der Sonde (3) im austauschbaren Innenrohr (105) angeordnet ist, wobei die Hülse (104) austauschbar ist und das Innenrohr (105) auf Position hält.

6. Vorrichtung nach einem der Patentansprüche 1-5, ferner **dadurch gekennzeichnet, dass** die Schulter zwischen dem vorderen Teil (31) und dem hinteren Teil (32) der Sonde (3) zu Beginn der Messung gegen eine Öffnung in der inneren Hülse (104) anliegt, die einen Anschlag für den hinteren Teil (32) der Sonde (3) aufnimmt.

7. Vorrichtung nach einem der Patentansprüche 1-6, ferner **gekennzeichnet durch** Mittel zur Korrektur von Messergebnissen, bei welcher Korrektur es sich um einen Algorithmus handelt.

8. Vorrichtung nach einem der Patentansprüche 1-7, ferner **gekennzeichnet durch** Mittel zur Korrektur von Messergebnissen proportional dazu, wie viel Bewegungsenergie beim Aufschlag und Rückprall der Sonde verloren geht.

## Revendications

1. Dispositif pour mesurer la pression intraoculaire, comprenant une sonde (3) soutenue et située dans un boîtier (1), une bobine avant (101) et une bobine arrière (102), et ou la bobine avant (101) ou la bobine arrière (102) a la faculté que donner à la sonde une vélocité spécifique, des moyens pour traiter et visualiser les données de mesures et contrôler l'opération, et un manchon interne (104) autour de la sonde (3), la sonde (3) étant formée d'une partie arrière (32) faite dans une matière magnétique et d'une partie avant (31), fixée à la partie arrière, faite dans une matière non magnétique et avec une pointe de forme ronde, de manière à ce que le joint entre ladite partie avant (31) et ladite partie arrière (32) soit située, au début de l'opération de mesure, à l'intérieur de la bobine (101) et la pointe de ladite partie avant (31) étant située à l'extérieur de la bobine avant (101), **caractérisé en ce que** le manchon interne (104) déborde sur une ouverture à travers laquelle la partie arrière passe (32), et **en ce que** le joint entre ladite partie avant (31) et ladite partie arrière (32) forment une épaule, qui repose sur les bords du manchon interne (104).

2. Dispositif selon la revendication 1, **caractérisé en ce qu'**une desdites bobines (101, 102) donnant à ladite sonde (3) une vélocité spécifique, fonctionne, quand le courant est branché audit dispositif, comme un dispositif de retenue maintenant ladite sonde en place.

3. Dispositif selon la revendication 1, **caractérisé en ce que** dans ledit boîtier (1) du dispositif, il y a, essentiellement après ladite sonde (3), un rétrécissement (10), qui permet de visualiser les parties de oeil qui sont mesurées, afin de garantir la précision.

4. Dispositif selon l'une quelconque des revendications 1 à 3, **caractérisé en**core en ce qu'il contient un support frontal (2) réglable (4).

5. Dispositif selon l'une quelconque des revendications 1 à 4, **caractérisé en ce qu'**il contient un tube interne remplaçable (105), ladite partie arrière (32) de ladite sonde (3) placée dans le tube interne remplaçable (105), le manchon interne (104) étant remplaçable et maintenant ledit tube interne (105) en place.

6. Dispositif selon l'une quelconque des revendications 1 à 5, **caractérisé en ce que** ladite épaule entre ladite partie avant (31) et ladite partie arrière (32) de ladite sonde (3) est située, au début de l'opération de mesure, contre une ouverture dans ledit manchon interne (104), qui reçoit le bord de ladite partie arrière (32) de ladite sonde (3).

7. Dispositif selon l'une quelconque des revendications 1 à 6, **caractérisé en ce qu'**il contient des moyens pour corriger les résultats de mesure, la correction étant un algorithme.

8. Dispositif selon l'une quelconque des revendications 1 à 7, **caractérisé en ce qu'**il contient des moyens pour corriger les résultats de mesure proportionnellement à la quantité d'énergie cinétique perdue lors de l'impact et du rebondissement de la sonde.
